# EUROPÄISCHE PATENTSCHRIFT

(11) **EP 1 400 250 B1**
(45) Veröffentlichungstag und Bekanntmachung des Hinweises auf die Patenterteilung: **28.02.2007**
(21) Anmeldenummer: 03016400.8
(22) Anmeldetag: 21.07.2003
(51) Int. Cl.: A61L 2/00, C12M 1/00

(54) **Sterilkolben und dessen Verwendung**
Sterile container and uses thereof
Conteneur stérile et ses applications

(30) Priorität: 18.09.2002 DE 10243331
(43) Veröffentlichungstag der Anmeldung: 24.03.2004
(73) Patentinhaber: InfraServ GmbH & Co. Höchst KG, 65926 Frankfurt am Main (DE)
(72) Erfinder: Simon, Klaus Jürgen, 65779 Kelkheim (DE); Itter, Klaus, 61184 Karben-Kloppenheim (DE)
(74) Vertreter: Ackermann, Joachim

(56) Entgegenhaltungen:
- DE-B- 1 063 336
- US-A- 3 532 605
- US-A- 5 130 093

## Beschreibung

Die vorliegende Erfindung betrifft einen Kolben, der sich besonders zur Durchführung von Reaktionen in steriler Umgebung eignet.

Herkömmliche Kolben für die Sterilproduktion bestehen üblicherweise aus einem Bodenteil, das mit dem Kolbenoberteil verschweißt ist. In der Sterilproduktion, beispielsweise bei der Herstellung pharmazeutischer Produkte oder Zwischenprodukte, ist es unerlässlich, dass die eingesetzten Apparaturen von Zeit zu Zeit sterilisiert werden. In den Schweißnähten herkömmlicher Kolben können sich in dort vorhandenen Lunkern oder Poren Produktionsrückstände ablagern, die den Kolbeninhalt in den darauf folgenden Produktionsschritten verunreinigen bzw. die die Sterilisation des Kolbens erschweren. Außerdem sind solche Kolben aufgrund ihrer Größe schwierig zu handhaben.

Mit der vorliegenden Erfindung wird ein Kolben vorgeschlagen, der einfach zu handhaben ist und bei dem das Risiko der Ablagerung von Rückständen aus der Produktion deutlich herabgesetzt ist.

Der erfindungsgemäße Kolben kann zerlegt und auf einfache Art und Weise sterilisiert und wieder zusammengesetzt werden.

Die vorliegende Erfindung betrifft einen Kolben (1 ) umfassend ein Oberteil in Form einer rotationssymmetrischen Mantelfläche (3), das eine Oberseite (4) und eine offene Unterseite (5) aufweist, wobei in der Unterseite (5) Dichtungsvorrichtungen (6) und Befestigungsvorrichtungen (7) vorgesehen sind, welche das Oberteil mit einer Bodenplatte (8) flüssigkeitsdicht verbinden, und wobei die Bodenplatte eine Ableitung (9) für den Kolbeninhalt aufweist.

Das Oberteil des Kolbens (1) weist die Form einer rotationssymmetrischen Mantelfläche (3) auf. Dabei kann es sich um die Mantelfläche eines Zylinders oder insbesondere eines Kegelstumpfes handeln. Es kann sich auch um den Teil einer Kugeloberfläche handeln, der auf der unteren Seite offen ist und an seiner Oberseite eine Einlassöffnung aufweist.

Auf der offenen Unterseite (5) sind Dichtungsvorrichtungen (6) und Befestigungsvorrichtungen (7) vorgesehen sind, welche das Oberteil mit einer Bodenplatte (8) flüssigkeitsdicht verbinden. Bei der Dichtungsvorrichtung (6) kann es sich um eine Dichtungsfläche handeln, die zusammen mit einer Dichtung, beispielsweise einem O-Ring, auf die Bodenplatte (8) aufgesetzt wird.

Bei der Befestigungsvorrichtung (7) kann es sich um eine beliebige Vorrichtung handeln, mit der Bodenplatte (8) und Oberteil miteinander verbunden werden können.

Beispiele dafür sind Schraub- oder Klemmverbindungen, wie Rohrverschraubungen (beispielsweise gemäß DIN 11864-1 ) oder aseptische Flanschverbindungen (beispielsweise gemäß DIN 11864-2)

In einer bevorzugten Ausführungsform sind Bodenplatte (8) und Befestigungsvorrichtung (7) als Flanschverbindung ausgestaltet. Dabei ist am äußeren Umfang der Unterseite (5) eine rotationssymmetrische Dichtungsfläche vorgesehen, in die bevorzugt eine Nut für die Aufnahme einer Dichtung eingefräst ist. Dichtungsfläche und Bodenplatte (8) werden vorzugsweise mit Schrauben miteinander verbunden.

Die Bodenplatte (8) des erfindungsgemäßen Kolbens (1 ) weist eine Ableitung (9) für den Kolbeninhalt auf.

Diese ist vorzugsweise in die Unterseite der Bodenplatte (8) eingearbeitet und steht mit dem Kolbeninhalt in Verbindung.

Vorzugsweise ist der Teil der Bodenplatte (8), die den Boden des Kolbens (1 ) bildet, als Schräge ausgestaltet, deren tiefster Punkt sich vorzugsweise gegenüberliegend zur Öffnung der Ableitung (9) in der Bodenplatte und unterhalb der Öffnung der Ableitung (9) in der Bodenplatte befindet. Dadurch wird eine definiert unvollständige Entleerung des Kolbeninhalts möglich.

In einer bevorzugten Ausführungsform des Kolbens ist die rotations-symmetrische Mantelfläche (3) in Form eines Kegelstumpfes (Trichters) ausgestaltet, dessen größerer Durchmesser die Unterseite (5) bildet.

Auf der Oberseite (4) dieser bevorzugten Ausführungsform befindet sich vorzugsweise eine Zuleitung (10) für den Kolbeninhalt. Diese kann abnehmbar ausgestaltet sein, und ebenfalls über eine Dichtungs- und Befestigungsvorrichtung mit der rotationssymmetrischen Mantelfläche (3) verbunden sein.

In einer weiteren bevorzugten Ausführungsform des erfindungsgemäßen Kolbens sind zumindest die rotationssymmetrische Mantelfläche (3) und die Befestigungsvorrichtungen (7) einerseits sowie die Bodenplatte (8) und die Ableitung (9) andererseits jeweils aus einem Teil gefertigt. In dieser Ausführungsform kann auf Schweißnähte ganz verzichtet werden, und die Reinigung und Sterilisation der Teile des Kolbens wird vereinfacht.

Die Erfindung betrifft auch die Verwendung des Kolbens als sterilisierbarer Impf- oder Schüttelkolben.

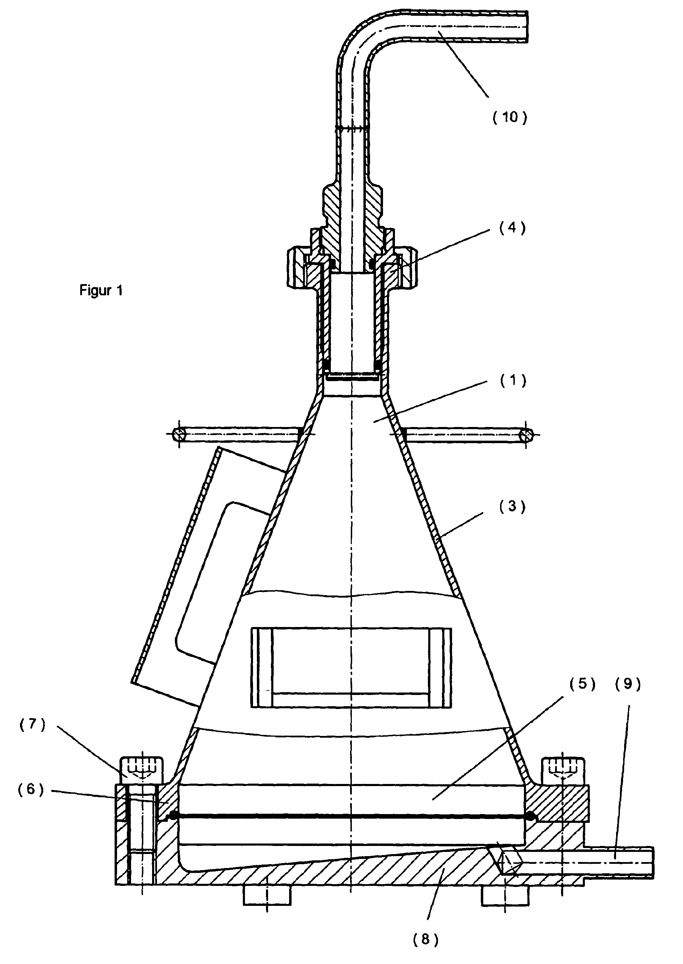

Die Figur erläutert den erfindungsgemäßen Kolben im Längsschnitt. Eine Beschränkung des Gegenstands der Erfindung ist dadurch nicht beabsichtigt.

Der Kolben (1) wird aus einem Oberteil in Form einer rotations-symmetrischen Mantelfläche (3) gebildet, der die Form eines Kegelstumpfes bzw. Trichters aufweist. Dessen Oberseite (4) ist mit einer Zuleitung (10) versehen. Die Unterseite (5) ist mit einem Flansch (6) versehen, der durch Schrauben (7) mit der Bodenplatte (8) verbunden ist. In die Bodenplatte (8) ist eine Ableitung (9) eingearbeitet, die mit dem Kolbeninhalt in Verbindung steht.

Der Teil der Bodenplatte (8), die den Boden des Kolbens (1 ) bildet, ist als Schräge ausgestaltet, deren tiefster Punkt sich gegenüberliegend zur Öffnung der Ableitung (9) in der Bodenplatte und unterhalb der Öffnung der Ableitung (9) in der Bodenplatte befindet.

Dadurch wird eine definiert unvollständige Entleerung des Kolbeninhalts möglich. Dieses kann beispielsweise von Vorteil sein, wenn der Kolbeninhalt nach Durchführung einer Reaktion einen bestimmten Gehalt von Mikroorganismen aufweist, und wenn nach Entleerung des Kolbens ein neuer Ansatz mit einem definierten Gehalt einer Impflösung durchgeführt werden soll.

Die Materialien der einzelnen Bestandteile des Kolbens unterliegen keinen besonderen Beschränkungen. Üblicherweise werden Oberteil und Bodenplatte (8) aus Metallen ausgeführt. Es sind aber auch Ausführungen aus Glas oder aus Kunststoff möglich. Bevorzugt bestehen alle Teile des erfindungsgemäßen Kolbens aus ein und demselben Material.

## Patentansprüche

1. Kolben (1) umfassend ein Oberteil in Form einer rotationssymmetrischen Mantelfläche (3), das eine Oberseite (4) und eine offene Unterseite (5) aufweist, wobei in der Unterseite (5) Dichtungsvorrichtungen (6) und Befestigungsvorrichtungen (7) vorgesehen sind, welche das Oberteil mit einer Bodenplatte (8) flüssigkeitsdicht verbinden, und wobei die Bodenplatte eine Ableitung (9) für den Kolbeninhalt aufweist.

2. Kolben nach Anspruch 1, **dadurch gekennzeichnet, dass** Bodenplatte (8) und Befestigungsvorrichtung (7) als Flanschverbindung ausgestaltet sind.

3. Kolben nach Anspruch 1, **dadurch gekennzeichnet, dass** die rotationssymmetrische Mantelfläche (3) in Form eines Trichters ausgestaltet ist, dessen grösserer Durchmesser die Unterseite (5) bildet.

4. Kolben nach Anspruch 3, **dadurch gekennzeichnet, dass** auf der Oberseite (4) eine Zuleitung (10) für den Kolbeninhalt vorgesehen ist.

5. Kolben nach Anspruch 1, **dadurch gekennzeichnet, dass** der Teil der Bodenplatte (8), die den Boden des Kolbens (1) bildet, als Schräge ausgestaltet ist, deren tiefster Punkt sich unterhalb der Öffnung der Ableitung (9) in der Bodenplatte befindet.

6. Kolben nach Anspruch 5, **dadurch gekennzeichnet, dass** der tiefste Punkt der Bodenplatte (8) gegenüberliegend zur Öffnung der Ableitung (9) in der Bodenplatte befindet.

7. Kolben nach Anspruch 1, **dadurch gekennzeichnet, dass** die rotationssymmetrische Mantelfläche (3) und die Befestigungsvorrichtungen (7) einerseits sowie die Bodenplatte (8) und die Ableitung (9) andererseits jeweils aus einem Teil gefertigt sind.

8. Verwendung des Kolbens nach Anspruch 1 als sterilisierbarer Impf- oder Schüttelkolben.

## Claims

1. A flask (1) comprising a top part which is in the form of a rotationally symmetrical jacket surface (3) and which has an upper side (4) and an open underside (5), the underside (5) being provided with sealing devices (6) and fastening devices (7) which connect the top part to a base plate (8) in a leaktight manner, the base plate having a drain line (9) for the contents of the flask.

2. The flask as claimed in claim 1, wherein the base plate (8) and fastening device (7) are designed as a flange connection.

3. The flask as claimed in claim 1, wherein the rotationally symmetrical jacket surface (3) is designed in the form of a funnel whose greater diameter forms the underside (5).

4. The flask as claimed in claim 3, wherein a delivery line (10) for the contents of the flask is provided on the upper side (4).

5. The flask as claimed in claim 1, wherein the part of the base plate (8) that forms the bottom of the flask (1) is designed as a slope whose lowest point is located below the opening of the drain line (9) in the base plate.

6. The flask as claimed in claim 5, wherein the lowest point of the base plate (8) is located lying opposite the opening of the drain line (9) in the base plate.

7. The flask as claimed in claim 1, wherein the rotationally symmetrical jacket surface (3) and fastening devices (7) on the one hand, and the base plate (8) and drain line (9) on the other, are each produced from one piece.

8. Use of the flask as claimed in claim 1 as a sterilizable inoculation flask or shaker flask.

## Revendications

1. Cône (1) comprenant une partie supérieure en forme d'enveloppe à symétrie de rotation (3), qui présente une face supérieure (4) et une face inférieure ouverte (5), des dispositifs d'étanchéité (6) et des dispositifs de fixation (7) sont prévus dans la face inférieure (5), lesquels rattachent la partie supérieure à une plaque de base (8) en assurant une étanchéité aux liquides, et la plaque de base présentant une dérivation (9) pour le contenu du cône.

2. Cône selon la revendication 1, **caractérisé en ce que** la plaque de base (8) et les dispositifs de fixation (7) sont conçus comme un raccordement à brides.

3. Cône selon la revendication 1, **caractérisé en ce que** l'enveloppe à symétrie de rotation (3) est conçue sous forme d'un entonnoir, dont le plus grand diamètre constitue la face inférieure (5).

4. Cône selon la revendication 3, **caractérisé en ce qu'**une conduite d'alimentation (10) pour le contenu du cône est prévue sur la face supérieure (4).

5. Cône selon la revendication 1, **caractérisé en ce que** la partie de la plaque de base (8) qui forme le fond du cône (1) présente une inclinaison dont le point le plus bas se trouve au-dessous de l'ouverture de la dérivation (9) dans la plaque de base.

6. Cône selon la revendication 5, **caractérisé en ce que** le point le plus bas de la plaque de base (8) se trouve à l'opposé de l'ouverture de la dérivation (9) dans la plaque de base.

7. Cône selon la revendication 1, **caractérisé en ce que** l'enveloppe à symétrie de rotation (3) et les dispositifs de fixation (7) d'une part ainsi que la plaque de base (8) et la dérivation (9) d'autre part sont chacun formés par une seule pièce.

8. Utilisation du cône selon la revendication 1 comme cône vibrant ou d'inoculation stérilisable.
